(19) Europäisches Patentamt
European Patent Office
Office européen des brevets

(11) EP 0 481 744 B1

(12) EUROPEAN PATENT SPECIFICATION

(45) Date of publication and mention of the grant of the patent:
**18.09.1996 Bulletin 1996/38**

(51) Int. Cl.[6]: **C07D 295/18**, A61K 31/55

(21) Application number: **91309513.9**

(22) Date of filing: **16.10.1991**

(54) **Piperazine derivatives**

Piperazinderivate

Dérivés de pipérazine

(84) Designated Contracting States:
**AT BE CH DE DK ES FR GR IT LI LU NL SE**

(30) Priority: **19.10.1990 GB 9022820**

(43) Date of publication of application:
**22.04.1992 Bulletin 1992/17**

(73) Proprietor: **JOHN WYETH & BROTHER LIMITED**
**Maidenhead Berkshire, SL6 0PH (GB)**

(72) Inventor: **Cliffe, Ian Anthony**
**Slough, Berkshire, SL1 5NT (GB)**

(74) Representative: **Brown, Keith John Symons et al**
**c/o Wyeth Laboratories**
**Huntercombe Lane South**
**Taplow Maidenhead Berkshire SL6 0PH (GB)**

(56) References cited:
**US-A- 2 776 282** **US-A- 4 921 958**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 481 744 B1

Printed by Rank Xerox (UK) Business Services
2.13.4/3.4

## Description

This invention relates to piperazine derivatives, to processes for their preparation, to their use and to pharmaceutical compositions containing them. The novel compounds act on the central nervous system by binding to 5-HT receptors (as more fully explained below) and hence can be used as medicaments for treating humans and other mammals.

R.A. Glennon, J. Med. Chem., 1987,30, 2-9 discusses central serotonin (5-HT) receptors as targets for drug research and discloses various arylpiperazines which act at the different 5-HT receptor sites.

US-A-4921958 discloses piperazinylalkyl-carboxylic acid adamantylamides some of which exhibit selective 5-$HT_{1A}$ receptor affinity and some of them which exhibit both 5-$HT_{1A}$ receptor affinity and dopamine D2 receptor binding.

The novel compounds of the invention are the compound of the formula

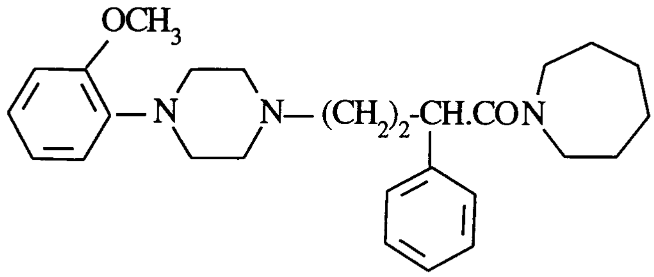

(A)

and the pharmaceutically acceptable salts thereof.

Compound A is 2,3,4,5,6,7-hexahydro-1-{4-[1-[4-(2-methoxyphenyl)-piperazinyl]]-2-phenylbutyryl}-1H-azepine.

Our copending U.K. application No. 9008925.1 describes compounds having a general formula which embraces the novel compounds of the present invention. The copending application corresponds to EP-A-395312 which was published after the priority date of the present invention. The compounds of our copending application are those of the general formula

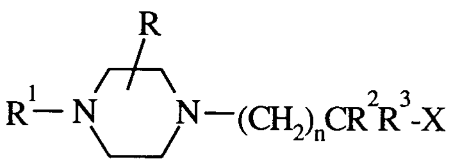

(I)

and the pharmaceutically acceptable acid addition salts thereof.

In formula (I)

n is one of the integers 1 or 2,
R is hydrogen or lower alkyl,
$R^1$ is an aryl or nitrogen containing heteroaryl radical,
$R^2$ is hydrogen or lower alkyl,
$R^3$ is an aryl radical, an alkyl radical containing 4 to 8 carbon atoms or an aryl(lower)alkyl radical,
X is $-OCOR^{10}$, $-CO_2R^6$, $-CONR^5R^9$, $-OCO_2R^6$, $-NR^4COR^6$, $OCONHR^{11}$, $-NHCO_2R^6$, $-NR^4CONHR^6$, $-CONHNHR^6$, $-CONHOR^6$,

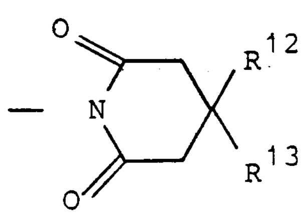

or

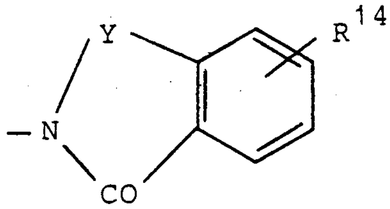

$R^4$ and $R^5$ are each hydrogen or lower alkyl

$R^6$ is $-CHR^7R^8$, cycloalkyl of 3 to 12 carbon atoms or aryl(lower)alkyl (where $R^7$ and $R^8$ are each hydrogen or lower alkyl),

$R^9$ is hydrogen, an alkyl group of 1 to 8 carbon atoms other than a tertiary alkyl group, cycloalkyl of 3 to 12 carbon atoms, cycloalkyl(lower)alkyl, aryl, aryl(lower)alkyl or 8-azaspiro[4.5]deca-7,9-dione-8-yl-(lower)alkyl [with the proviso that when $R^3$ is aryl or aralkyl, $R^9$ is not a phenyl group substituted in the ortho position by halogen, nitro, trifluoroalkyl, cyano, sulphonic acid, sulphonamido, carboxy, carbalkoxy, carboxylanilino or a 4-carboxylaminobenzosulphonamido group and that when $R^9$ is hydrogen,alkyl, aryl or aryl(lower)alkyl $R^5$ is hydrogen or $-CHR^7R^8$], or $R^5$ and $R^9$ together with the nitrogen atom to which they are attached represent an azetidino, pyrrolidino, piperidino, hexahydroazepino, morpholino or piperazino ring which may be optionally substituted by lower alkyl, aryl or aryl(lower)alkyl

$R^{10}$ is cycloalkyl of 3 to 12 carbon atoms, or 2,3-dihydro[1,4]benzodioxinyl optionally substituted by lower alkyl, lower alkoxy or halogen or, when R3 is an alkyl radical containing 4 to 8 carbon atoms, $R^{10}$ can also be aryl;

$R^{11}$ is cycloalkyl of 3 to 12 carbon atoms, aryl or aryl(lower)alkyl,

$R^{12}$ and $R^{13}$ are each lower alkyl or together with the carbon atom to which they are both attached represent $C_{4-6}$ cycloalkyl,

$R^{14}$ represents hydrogen, halogen, lower alkyl or lower alkyl

and

Y is CO or $SO_2$.

The compounds of our copending application can be prepared by a number of methods from known starting materials or starting materials that may be prepared by conventional methods. Some of the disclosed methods are given below:

In one method for preparing an amide of formula (I), where X represents $-CONR^5R^9$ an amine of formula

$$NHR^5R^9 \qquad (II)$$

where $R^5$ and $R^9$ are as defined above is acylated with an acid of formula

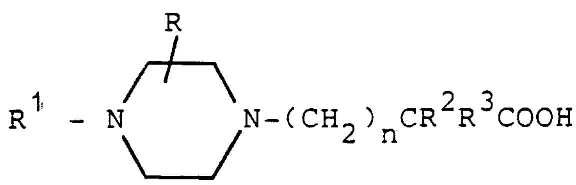

(III)

(where R, $R^1$, $R^2$ and $R^3$ are as defined above) or with an acylating derivative thereof. Examples of acylating derivatives include the acid halides (eg acid chlorides), azides, anhydrides, imidazolides (eg obtained from carbonyldiimidazole), activated esters or O-acyl ureas obtained from a carbodiimide such as a dialkylcarbodiimide particularly dicyclohexylcarbodiimide. Preferably the amine is acylated with the acid in presence of a coupling agent such as 1,1′-carbonyldiimidazole, iso-butylchloroformate or diphenylphosphinyl chloride.

An alternative method of preparing the compounds of formula (I) comprises alkylation of a piperazine of formula

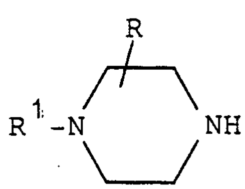

(IV)

(where R and $R^1$ are as defined above) with an alkylating agent providing the group

$$-(CH_2)_nCR^2R^3X \qquad (V)$$

(where n, $R^2$, $R^3$ and X are as defined above).

The alkylating agent may be, for example, a compound of formula

$$Z\text{-}CH_2CR^2R^3X \qquad \text{(VI)}$$

where $R^2$, $R^3$ and X are as defined above and Z is a leaving group such as halogen or an alkyl- or aryl-sulphonyloxy group. Alternatively the alkylating agent may be an unsaturated compound of formula

$$CH_2{=}CR^3X \qquad \text{(VII)}$$

(where $R^3$ and X are as defined above) and the compound of formula (VII) is reacted with the piperazine of formula (IV) by means of a Michael reaction. The reaction may be carried out at elevated temperature in the presence of an alcohol. A small quantity of an acid catalyst may be employed in the reaction when X represents $-CONR^5R^9$.

The compound of the invention of formula A may be prepared in an analogous manner using appropriate starting materials. A preferred method of preparing compound A comprises reacting a compound of formula

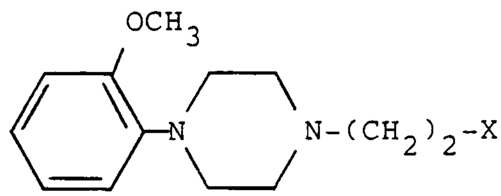

where X is a leaving group, such as halogen, with an anion of the amide of formula

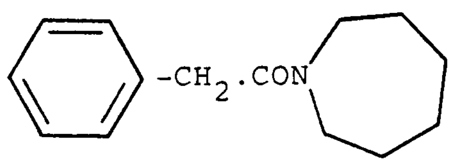

The anion may be prepared by reacting the amide with a strong base, eg lithium diisopropylamide.

The processes described above may be carried out to give a compound of the invention in the form of a free base or as an acid addition salt. If the compound of the invention is obtained as an acid addition salt, the free base can be obtained by basifying a solution of the acid addition salt. Conversely, if the product of the process is a free base an acid addition salt, particularly a pharmaceutically acceptable acid addition salt, may be obtained by dissolving the free base in a suitable organic solvent and treating the solution with an acid, in accordance with conventional procedures for preparing acid addition salts from base compounds.

Examples of acid addition salts are those formed from inorganic and organic acids, such as sulphuric, hydrochloric, hydrobromic, phosphoric, tartaric, fumaric, maleic, citric, acetic, formic, methanesulphonic, p-toluenesulphonic, oxalic and succinic acids.

The compounds of the invention contain an asymmetric carbon atom, so that the compounds can exist in different steroisomeric forms. The compounds can be, for example, racemates or optically active forms. The optically active forms can be obtained by resolution of the racemates or by asymmetric synthesis. For example resolution may be carried out by forming diastereoisomeric salts of the racemic base with an optically active acid (eg dibenzoyl-L-tartaric acid), separating the diastereoisomeric salts and converting them to the optically active base or other salts.

The compounds of the present invention possess pharmacological activity. In particular, they act on the central nervous system by binding to 5-HT receptors. In pharmacological testing it has been shown that the compounds particularly bind to receptors of the $5\text{-}HT_{1A}$ type. The compounds selectively bind to receptors of the $5\text{-}HT_{1A}$ type to a much greater extent than they bind to other receptors such as $\alpha_1$ receptors. They exhibit activity as $5\text{-}HT_{1A}$ antagonists in pharmacological testing. The pharmacological testing of the compounds indicates that they can be used for the treatment of CNS disorders, such as anxiety in mammals, particularly humans. They may also be useful as antidepressants, hypotensives and as agents for regulating the sleep/wake cycle, feeding behaviour and/or sexual function.

The compounds of the invention were tested for $5\text{-}HT_{1A}$ receptor binding activity in rat hippocampal membrane homogenate by the method of B S Alexander and M D Wood, J Pharm Pharmacol, 1988, 40, 888-891.

The results indicate that the compounds of the invention are more potent than other compounds of formula I including the related compound 2,3,4,5,6,7-hexahydro-1-{3-{1-[4-(2-methoxyphenyl)piperazinyl]}-2-phenylpropionyl}-1H-azepine (compound B) described in Example 34 of our copending application. Compound B was one of the most potent

compounds disclosed in the copending application. The results are given below, in which compound C is the isomer described in Example 2(a) below:

| | $IC_{50}$ (nM) |
|---|---|
| Compound A | 3 |
| Compound B | 9 |
| Compound C | 1 |

The compounds were also tested for $5HT_{1A}$ receptor antagonism activity in a test involving the antagonism of 8-hydroxy-2-(di-n-propylamino)-tetralin (8-OH DPAT) syndrome in the rat. The results are given below

| | MED (mg/kg; s.c.) |
|---|---|
| Compound A | 0.03 |
| Compound B | 0.3 |

The compounds were also tested for potential anxiolytic activity by a test procedure measuring mouse exploratory activity in a two-compartment light/dark box based upon the procedure of B Costall et al, Neuropharmacology, 1987, 26, 195-200 and J.N. Crawley et al, Pharmac. Biochem. Behav. 1980, 13, 167-170. The results are given below

| | MED (mg/kg; s.c.) |
|---|---|
| Compound A | 0.03 |
| Compound B | 1 |

The invention also provides a pharmaceutical composition comprising a compound of formula A or a pharmaceutically acceptable acid addition salt thereof in association with a pharmaceutically acceptable carrier. Any suitable carrier known in the art can be used to prepare the pharmaceutical composition. In such a composition, the carrier is generally a solid or liquid or a mixture of a solid or liquid.

Solid form compositions include powders, granules, tablets, capsules (eg hard and soft gelatine capsules), suppositories and pessaries. A solid carrier can be, for example, one or more substances which may also act as flavouring agents, lubricants, solubilisers, suspending agents, fillers, glidants, compression aides, binders or tablet-disintegrating agents; it can also be an encapsulating material. In powders the carrier is a finely divided solid which is in admixture with the finely divided active ingredient. In tablets the active ingredient is mixed with a carrier having the necessary compression properties in suitable proportions and compacted in the shape and size desired. The powders and tablets preferably contain up to 99%, eg from 0.03 to 99%, preferably 1 to 80% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

The term "composition" is intended to include the formulation of an active ingredient with encapsulating material as carrier to give a capsule in which the active ingredient (with or without other carriers) is surrounded by the carrier, which is thus in association with it. Similarly cachets are included.

Liquid form compositions include, for example, solutions, suspensions, emulsions, syrups, elixirs and pressurised compositions. The active ingredient, for example, can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilisers, emulsifiers, buffers, preservatives, sweeteners, flavouring agents, suspending agents, thickening agents, colours, viscosity regulators, stabilisers or osmoregulators. Suitable examples of liquid carriers for oral and parenteral administration include water (particularly containing additives as above, eg cellulose derivatives, preferably sodium carboxymethyl cellulose solution), alcohols, eg glycerol and glycols) and their derivatives, and oils (eg fractionated coconut oil and arachis oil). For parenteral

administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. When the compound is orally active it can be administered orally either in liquid or solid composition form.

Preferably the pharmaceutical composition is in unit dosage form, eg as tablets or capsules. In such form, the composition is sub-divided in unit dose containing appropriate quantities of the active ingredient; the unit dosage forms can be packaged composition, for example racketed powders, vials, ampoules, prefilled syringes or sachets containing liquid. The unit dosage form can be, for example, a capsule or tablet itself, or it can be the appropriate number of any such compositions in package form. The quantity of the active ingredient in unit dose of composition may be varied or adjusted from 0.5 mg or less to 750 mg or more, according to the particular need and the activity of the active ingredient.

The following Examples illustrate the invention:

Example 1

2,3,4,5,6,7-Hexahydro-1-{4-[1-[4-(2-methoxyphenyl)piperazinyl]]-2-phenylbutyryl}-1H-azepine

Butyl lithium (1.5M in hexane: 5 ml, 7.5 mmol) was added dropwise over 5 min, maintaining the temperature below 8°C, to a stirred solution of 2,3,4,5,6,7-hexahydro-1-phenylacetyl-1H-azepine (1.48 g, 6.8 mmol) and diisopropylamine (2.0 ml, 1.4 g, 14 mmol) in dry toluene (16 ml) under argon. The mixture was stirred at 0°C for 1 h, and a solution of freshly chromatographed 1-(2-chloroethyl)-4-(2-methoxyphenyl)piperazine (1.73 g, 6.8 mmol) in dry toluene (4 ml) was added dropwise. The mixture was stirred at 0°C to 20°C for 18 h, and water (50 ml) was added. The layers were separated, and the aqueous phase was extracted with ethyl acetate (2 x 50 ml). The combined organic phases were concentrated in vacuo. The crude product (2.91 g) was chromatographed on silica with eluant ethyl acetate to give the title compound as the free base (0.15 g). The product was dissolved in ethyl acetate (30 ml), and the solution was acidified with ethereal hydrogen chloride. The product was collected to give the title compound as the dihydrochloride three quarters hydrate (0.36 g), m.p. 175°-178°C (Found: C, 62.05; H, 7.8; N, 7.75. $C_{27}H_{37}N_3O_2$.2HCl.0.75 $H_20$ requires C, 62.1; H, 7.8; N, 8.05%).

Example 2

Resolution of 2,3,4,5,6,7-hexahydro-1-{4-[1-[4-(2-methoxyphenyl)piperazinyl]]-2-phenylbutyryl}-1H-azepine

(a) 2,3,4,5,6,7-Hexahydro-1-{4-[1-[4-(2-methoxyphenyl)-piperazinyl]]-2-phenylbutyryl}-1H-azepine (12.1 g) was dissolved in ethyl acetate (2.5 volumes, ie 30 ml) and a solution of dibenzoyl-L-tartaric acid monohydrate (1 mol eq) in ethyl acetate (2.5 volumes) added. The initially formed oil was redissolved by the addition of the minimum quantity of acetonitrile (3.6 ml). After 3 d, filtration gave crystals (6.2 g) which had an optical purity of 28% as judged by chiral H.P.L.C.. Recrystallisation first from ethyl acetate - acetonitrile (3:10, 13 volumes), which gave a sample with an optical purity of 84%, and secondly from methanol (7.5 volumes) gave the first enantiomer (isomer I) of the product as a dibenzoyl-L-tartrate (2.1 g) m.p. 147-150°C, $[\alpha]_D^{22}$ = -26° (1% in MeOH) (Found: C, 66.7; H, 6.7; N, 5.1 $C_{27}H_{37}N_3O_2$. $C_{18}H_{14}O_8$.$H_2O$ requires C, 66.6; H, 6.6; N, 5.2%) with an optical purity of 97.4%. The sample was converted to the free base of isomer (I) (1.1 g), $[\alpha]_D^{26}$ = +53° (1% in $CHCl_3$) and thence to the hydrochloride salt in the usual manner to afford a colourless powder (0.65 g), m.p. 181-184°C, $[\alpha]_D^{23}$ = +35° (1% in MeOH) (Found: C, 63.1; H, 7.8; N, 7.9. $C_{27}H_{37}N_3O_2$.2HCl.0.25$H_2O$ requires C, 63.2; H, 7.7; N, 8.2%) with an optical purity of 97.6%.

(b) The second enantiomer (isomer II) was formed in a similar manner from the racemate of Example 1 and dibenzoyl-D-tartaric acid monohydrate. Isomer II dibenzoyl-D-tartrate m.p. 141-142°C, $[\alpha]_D^{25}$ = +25° (1% in MeOH) (Found: C, 67.3; H, 6.7; N, 5.2. $C_{27}H_{37}N_3O_2$. $C_{18}H_{14}O_8$.0.25$H_20$ requires C, 67.3; H, 6.5; N, 5.2%). Isomer II base: $[\alpha]_D^{26}$ = -62° (1% solution in $CHCl_3$) Isomer II hydrochloride m.p. 181-184°C, $[\alpha]_D^{26}$ = -36° (1% in MeOH) (Found: C, 60.2; H, 7.7; N, 7.75 $C_{27}H_{37}N_3O_2$. 2HCl.1.75$H_20$ requires C, 60.05; H, 7.9; N, 7.8%) with an optical purity of 98.2%.

## Claims

**Claims for the following Contracting States : AT, BE, CH, DE, DK, FR, IT, LI, LU, NL, SE**

1. 2,3,4,5,6,7-Hexahydro-1-{4-[1-[4-(2-methoxyphenyl)piperazinyl]]-2-phenylbutyryl}-1H-azepine or a pharmaceutically acceptable acid addition salt thereof.

**2.** A compound as claimed in claim 1 which is the enantiomer of 2,3,4,5,6,7-hexahydro-1-{4-[1-[4-(2-methoxyphenyl)piperazinyl]]-2-phenylbutyryl}-1H-azepine in which the free base has the $[\alpha]_D^{26}$ of about +53° (1% in $CHCl_3$), or a pharmaceutically acceptable salt thereof.

**3.** A compound as claimed in claim 1 which is the enantiomer of 2,3,4,5,6,7-hexahydro-1-{4-[1-[4-(2-methoxyphenyl)piperazinyl]]-2-phenylbutyryl}-1H-azepine in which the free base has the $[\alpha]_D^{26}$ of about -62° (1% in $CHCl_3$), or a pharmaceutically acceptable salt thereof.

**4.** A process for preparing a compound claimed in claim 1 which comprises

(a) acylating an amine of formula

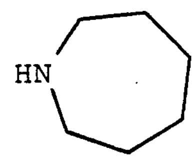

with an acid of formula

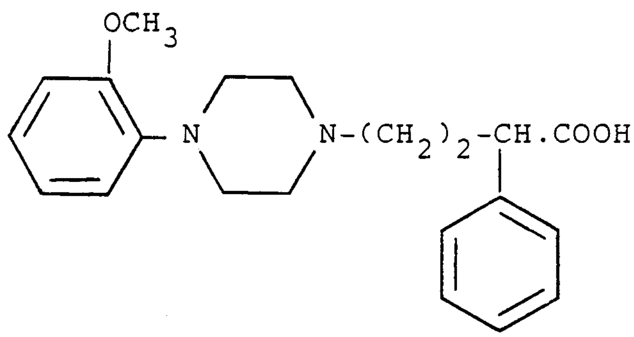

or with an acylating derivative thereof
or
(b) alkylating a piperazine of formula

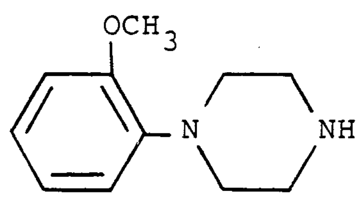

with an alkylating agent providing the group

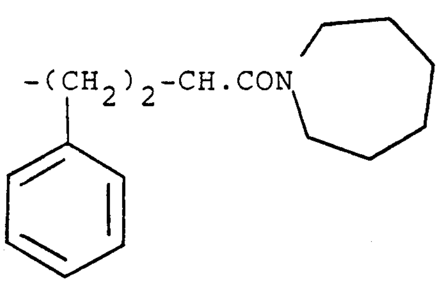

or

(c) reacting a compound of formula

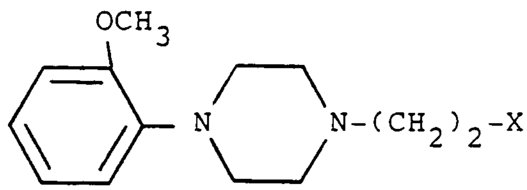

(where X is a leaving group) with an anion of the amide of formula

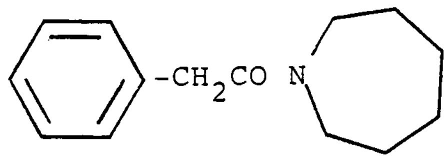

or

(d) converting a base claimed in claim 1 into a pharmaceutically acceptable acid addition salt thereof

or

(e) converting a pharmaceutically acceptable acid addition salt claimed in claim 1 into a free base

or

(f) resolving a racemic compound claimed in claim 1.

**5.** A pharmaceutical composition comprising a compound claimed in claim 1 in association with a pharmaceutically acceptable carrier.

**6.** A compound claimed in any one of claims 1,2 and 3 for use as a pharmaceutical.

**7.** A compound claimed in any one of claims 1,2 and 3 for use as an anxiolytic, an antidepressant, a hypotensive or as an agent for regulating the sleep/wake cycle, feeding behaviour and/or sexual function.

**Claims for the following Contracting States : ES, GR**

**1.** A process for preparing 2,3,4,5,6,7-hexahydro-1-[4-[1-[4-(2-methoxyphenyl)piperazinyl]]-2-phenylbutyryl]-1H-azepine or a pharmaceutically acceptable acid addition salt thereof which process comprises

(a) acylating an amine of formula

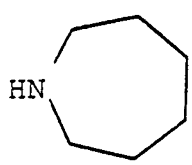

with an acid of formula

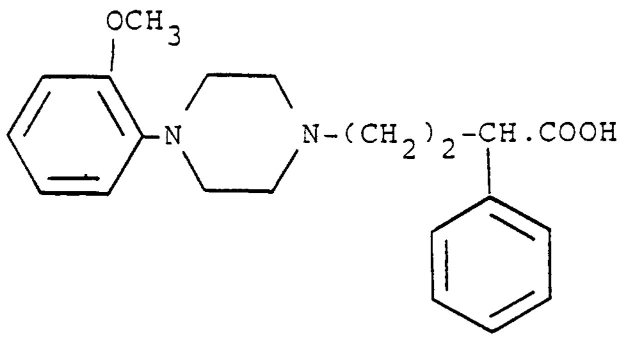

or with an acylating derivative thereof

or

(b) alkylating a piperazine of formula

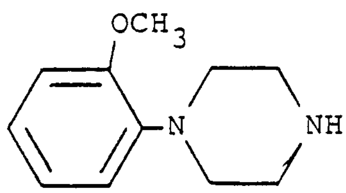

with an alkylating agent providing the group

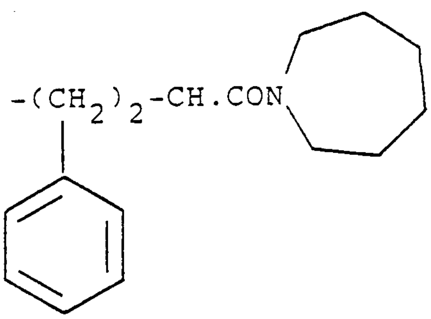

or

(c) reacting a compound of formula

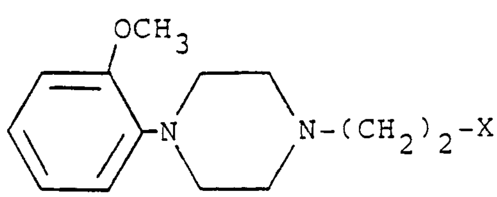

(where X is a leaving group) with an anion of the amide of formula

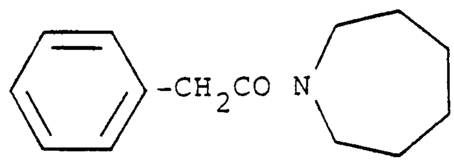

or

(d) converting the free base 2,3,4,5,6,7-hexahydro-1-{4-[1-[4-(2-methoxyphenyl)piperazinyl]]-2-phenylbutyryl}-1H-azepine into a pharmaceutically acceptable acid addition salt thereof

or

(e) converting a pharmaceutically acceptable acid addition salt of 2,3,4,5,6,7-hexahydro-1-{4-[1-[4-(2-methoxyphenyl)piperazinyl]]-2-phenylbutyryl}-1H-azepine into its free base

or

(f) resolving racemic 2,3,4,5,6,7-hexahydro-1-{4-[1-[4-(2-methoxyphenyl)piperazinyl]]-2-phenylbutyryl}-1H-azepine.

2. A process as claimed in claim 1 in which the product is the enantiomer of 2,3,4,5,6,7-hexahydro-1-{4-[1-[4-(2-methoxyphenyl)piperazinyl]]-2-phenylbutyryl}-1H-azepine in which the free base has the $[\alpha]_D^{26}$ of about +53° (1% in $CHCl_3$), or a pharmaceutically acceptable salt thereof.

3. A process as claimed in claim 1 in which the product is the enantiomer of 2,3,4,5,6,7-hexahydro-1-{4-[1-[4-(2-methoxyphenyl)piperazinyl]]-2-phenylbutyryl}-1H-azepine in which the free base has the $[\alpha]_D^{26}$ of about -62° (1% in $CHCl_3$), or a pharmaceutically acceptable salt thereof.

4. A process for preparing a pharmaceutical composition comprising bringing 2,3,4,5,6,7-hexahydro-1-{4-[1-[4-(2-methoxyphenyl)piperazinyl]]-2-phenylbutyryl}-1H-azepine or a pharmaceutically acceptable salt thereof into association with a pharmaceutically acceptable carrier.

5. A process as claimed in claim 4 in which the active ingredient is prepared by the process claimed in any one of claims 1 to 3.

**Patentansprüche**

**Patentansprüche für folgende Vertragsstaaten : AT, BE, CH, DE, DK, FR, IT, LI, LU, NL, SE**

1. 2,3,4,5,6,7-Hexahydro-1-{4-[1-[4-(2-methoxyphenyl)-piperazinyl]]-2-phenylbutyryl}-1H-azepin oder ein pharmazeutisch annehmbares Säureadditionssalz hievon.

2. Verbindung nach Anspruch 1, welche das Enantiomer von 2,3,4,5,6,7-Hexahydro-1-{4-[1-[4-(2-methoxyphenyl)-piperazinyl]]-2-phenylbutyryl}-1H-azepin, wobei die freie Base einen $[\alpha]_D^{26}$-Wert von etwa +53° (1 % in $CHCl_3$) aufweist, oder ein pharmazeutisch annehmbares Salz hievon ist.

3. Verbindung nach Anspruch 1, welche das Enantiomer von 2,3,4,5,6,7-Hexahydro-1-{4-[1-[4-(2-methoxyphenyl)-piperazinyl]]-2-phenylbutyryl}-1H-azepin, wobei die freie Base einen $[\alpha]_D^{26}$-Wert von etwa -62° (1 % in $CHCl_3$) aufweist, oder ein pharmazeutisch annehmbares Salz hievon ist.

4. Verfahren zur Herstellung einer Verbindung nach Anspruch 1, welches umfaßt:

(a) Acylieren eines Amins der Formel

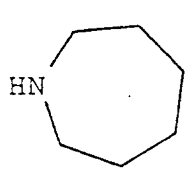

mit einer Säure der Formel

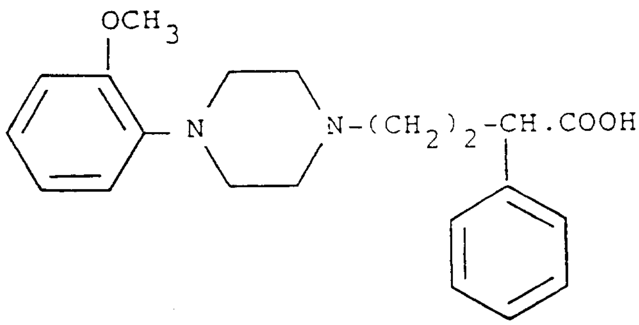

oder mit einem Acylierungs-Derivat hievon, oder

(b) Alkylieren eines Piperazins der Formel

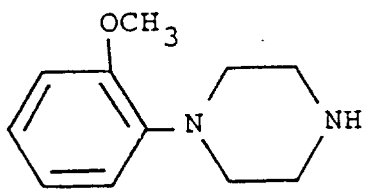

mit einem Alkylierungsmittel, welches die Gruppe

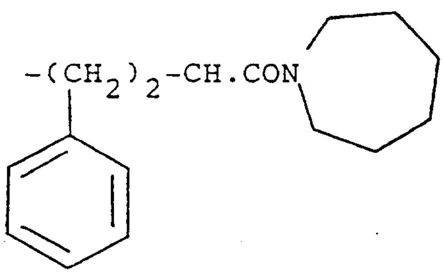

vorsieht, oder

(c) Umsetzen einer Verbindung der Formel

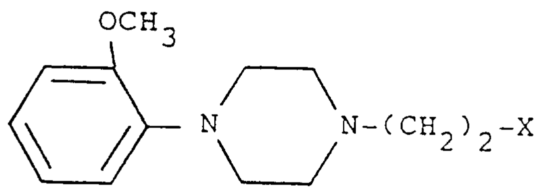

(worin X eine Abgangsgruppe bedeutet) mit einem Anion des Amids der Formel

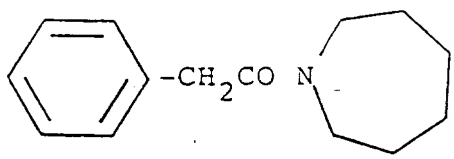

oder

(d) Überführen einer Base nach Anspruch 1 in ein pharmazeutisch annehmbares Säureadditionssalz hievon, oder

(e) Überführen eines pharmazeutisch annehmbaren Säureadditionssalzes nach Anspruch 1 in eine freie Base, oder

(f) Trennen einer racemischen Verbindung nach Anspruch 1.

**5.** Pharmazeutische Zusammensetzung, welche eine Verbindung nach Anspruch 1 in Vereinigung mit einem pharmazeutisch annehmbaren Träger umfaßt.

**6.** Verbindung nach einem der Ansprüche 1, 2 und 3 zur Verwendung als Pharmazeutikum.

**7.** Verbindung nach einem der Ansprüche 1, 2 und 3 zur Verwendung als Anxiolytikum, Antidepressivum, Hypotensivum oder als Mittel zur Regulierung des Schlaf/Wach-Zyklus, des Eßverhaltens und/oder der Sexualfunktion.

**Patentansprüche für folgende Vertragsstaaten : ES, GR**

**1.** Verfahren zur Herstellung von 2,3,4,5,6,7-Hexahydro-1-{4-[1-[4-(2-methoxyphenyl)-piperazinyl]]-2-phenylbutyryl}-1H-azepin oder eines pharmazeutisch annehmbaren Säureadditionssalzes hievon, welches Verfahren umfaßt:

(a) Acylieren eines Amins der Formel

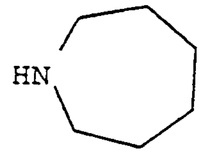

mit einer Säure der Formel

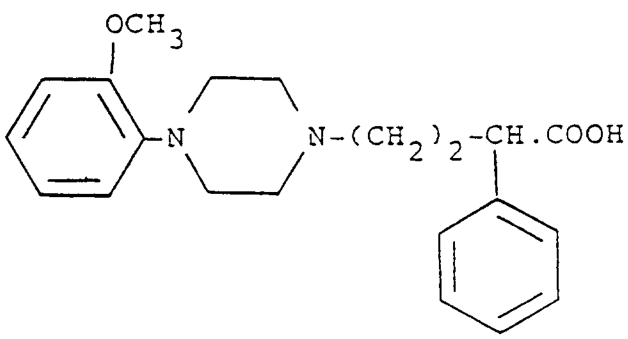

oder mit einem Acylierungs-Derivat hievon, oder

(b) Alkylieren eines Piperazins der Formel

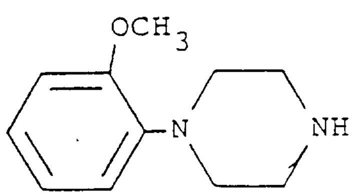

mit einem Alkylierungsmittel, welches die Gruppe

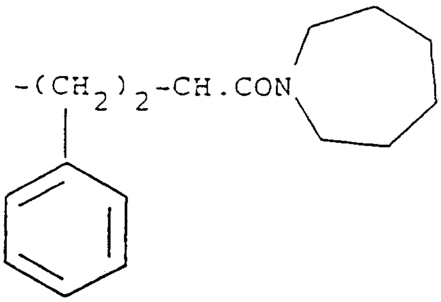

vorsieht, oder

(c) Umsetzen einer Verbindung der Formel

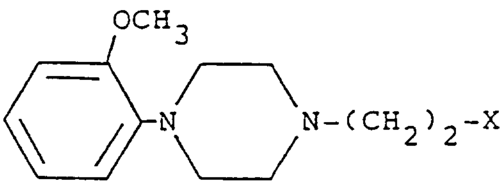

(worin X eine Abgangsgruppe bedeutet) mit einem Anion des Amids der Formel

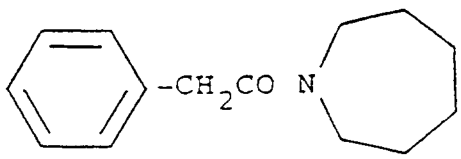

oder

(d) Überführen der freien Base 2,3,4,5,6,7-Hexahydro-1-{4-[1-[4-(2-methoxyphenyl)-piperazinyl]]-2-phenylbutyryl}-1H-azepin in ein pharmazeutisch annehmbares Säureadditionssalz hievon, oder

(e) Überführen eines pharmazeutisch annehmbaren Säureadditionssalzes von 2,3,4,5,6,7-Hexahydro-1-{4-[1-[4-(2-methoxyphenyl)-piperazinyl]]-2-phenylbutyryl}-1H-azepin in seine freie Base, oder

(f) Trennen von racemischem 2,3,4,5,6,7-Hexahydro-1-{4-[1-[4-(2-methoxyphenyl)-piperazinyl]]-2-phenylbutyryl}-1H-azepin.

2. Verfahren nach Anspruch 1, bei welchem das Produkt das Enantiomer von 2,3,4,5,6,7-Hexahydro-1-{4-[1-[4-(2-methoxyphenyl)-piperazinyl]]-2-phenylbutyryl}-1H-azepin, wobei die freie Base einen $[\alpha]_D^{26}$-Wert von etwa +53° (1 % in $CHCl_3$) aufweist, oder ein pharmazeutisch annehmbares Salz hievon ist.

3. Verfahren nach Anspruch 1, bei welchem das Produkt das Enantiomer von 2,3,4,5,6,7-Hexahydro-1-{4-[1-[4-(2-methoxyphenyl)-piperazinyl]]-2-phenylbutyryl}-1H-azepin, wobei die freie Base einen $[\alpha]_D^{26}$-Wert von etwa -62° (1 % in $CHCl_3$) aufweist, oder ein pharmazeutisch annehmbares Salz hievon ist.

4. Verfahren zur Herstellung einer pharmazeutischen Zusammensetzung, welches das Vereinigen von 2,3,4,5,6,7-Hexahydro-1-{4-[1-[4-(2-methoxyphenyl)-piperazinyl]]-2-phenylbutyryl}-1H-azepin oder eines pharmazeutisch annehmbaren Salzes hievon mit einem pharmazeutisch annehmbaren Träger umfaßt.

5. Verfahren nach Anspruch 4, bei welchem der aktive Bestandteil durch das Verfahren nach einem der Ansprüche 1 bis 3 hergestellt wird.

## Revendications

**Revendications pour les Etats contractants suivants : AT, BE, CH, DE, DK, FR, IT, LI, LU, NL, SE**

1. 2,3,4,5,6,7-Hexahydro-1-[4-[1-[4-(2-méthoxyphényl)pipérazinyl]]-2-phénylbutyryl]-1H-azépine ou un sel d'addition d'acide pharmaceutiquement acceptable de celle-ci.

2. Composé suivant la revendication 1, qui est l'enantiomère de la 2,3,4,5,6,7-hexahydro-1-[4-[1-[4-(2-méthoxyphényl)pipérazinyl]]-2-phénylbutyryl]-1H-azépine, pour lequel la base libre a un $[\alpha]_D^{26}$ d'environ +53° (1% dans le $CHCl_3$), ou un sel pharmaceutiquement acceptable de celui-ci.

3. Composé suivant la revendication 1, qui est l'énantiomère de la 2,3,4,5,6,7-hexahydro-1-[4-[1-[4-(2-méthoxyphényl)pipérazinyl]]-2-phénylbutyryl]-1H-azépine, pour lequel la base libre a un $[\alpha]_D^{26}$ d'environ -62° (1% dans le $CHCl_3$), ou un sel pharmaceutiquement acceptable de celui-ci.

4. Procédé de préparation d'un composé suivant la revendication 1, qui comprend :

   (a) l'acylation d'une amine de formule :

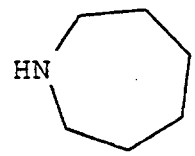

   avec un acide de formule :

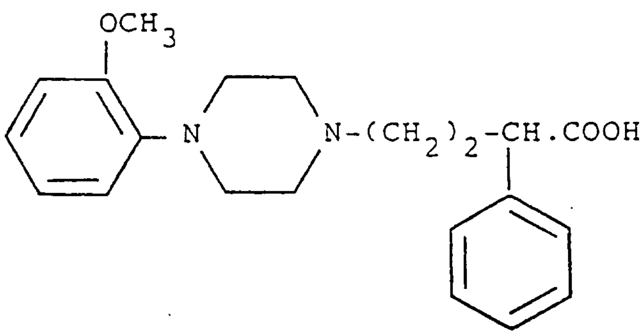

   ou avec un dérivé acylant de celui-ci, ou
   (b) l'alcoylation d'une pipérazine de formule :

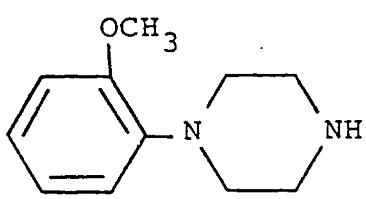

   avec un agent alcoylant fournissant le groupe :

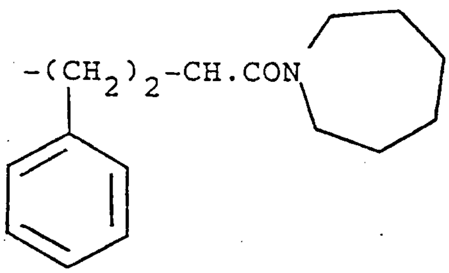

ou

(c) la réaction d'un composé de formule :

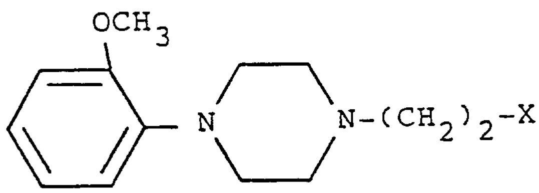

(où X est un groupe partant), avec un anion de l'amide de formule :

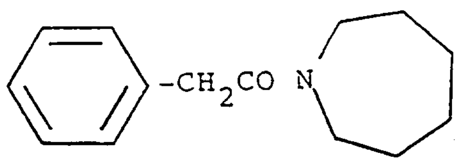

ou

(d) la conversion d'une base suivant la revendication 1, en un sel d'addition d'acide pharmaceutiquement acceptable de celle-ci, ou

(e) la conversion d'un sel d'addition d'acide pharmaceutiquement acceptable suivant la revendication 1 en une base libre, ou

(f) la résolution d'un composé racémique suivant la revendication 1.

**5.** Composition pharmaceutique comprenant un composé suivant la revendication 1, en association avec un excipient pharmaceutiquement acceptable.

**6.** Composé suivant l'une quelconque des revendications 1, 2 et 3, à utiliser comme produit pharmaceutique.

**7.** Composé suivant l'une quelconque des revendications 1, 2 et 3, à utiliser comme anxiolytique, antidépresseur, hypotenseur ou comme agent de régulation du cycle sommeil/éveil, du comportement alimentaire et/ou de la fonction sexuelle.

**Revendications pour les Etats contractants suivants : GR, ES**

**1.** Procédé de préparation de la 2,3,4,5,6,7-hexahydro-1-[4-[1-[4-(2-méthoxyphényl)pipérazinyl]]-2-phénylbutyryl]-1H-azépine ou d'un sel d'addition d'acide pharmaceutiquement acceptable de celle-ci, le procédé comprenant :

(a) l'acylation d'une amine de formule :

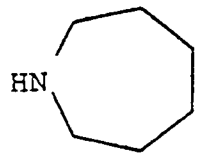

avec un acide de formule :

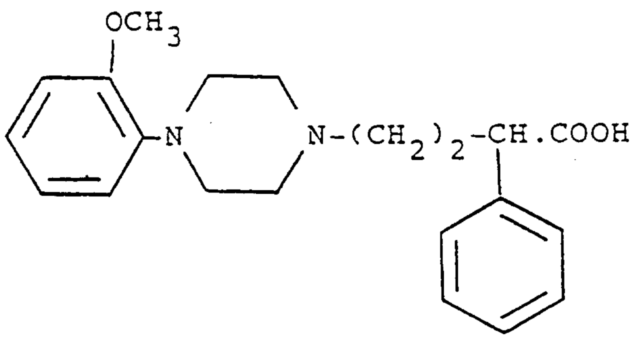

ou avec un dérivé acylant de celui-ci, ou

(b) l'alcoylation d'une pipérazine de formule :

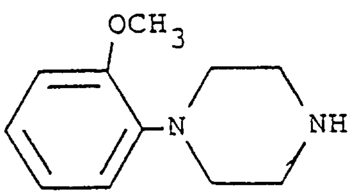

avec un agent alcoylant fournissant le groupe :

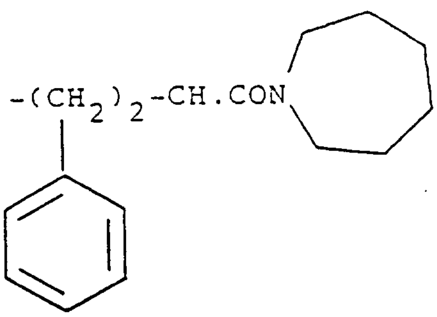

ou

(c) la réaction d'un composé de formule :

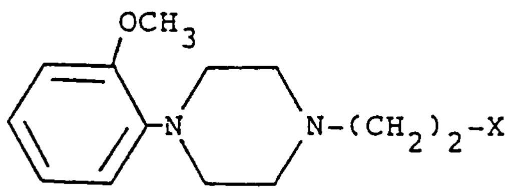

(où X est un groupe partant), avec un anion de l'amide de formule :

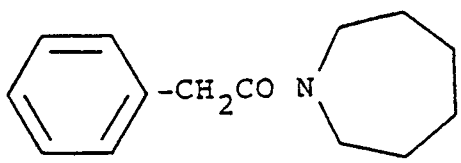

ou

(d) la conversion de la base libre 2,3,4,5,6,7-hexahydro-1-[4-[1-[4-(2-méthoxyphényl)pipérazinyl]]-2-phénylbutyryl]-1H-azépine en un sel d'addition d'acide pharmaceutiquement acceptable de celle-ci, ou

(e) la conversion d'un sel d'addition d'acide pharmaceutiquement acceptable de la 2,3,4,5,6,7-hexahydro-1-[4-[1-[4-(2-méthoxyphényl)pipérazinyl]]-2-phénylbutyryl]-1H-azépine en sa base libre, ou

(f) la résolution de la 2,3,4,5,6,7-hexahydro-1-[4-[1-[4-(2-méthoxyphényl)pipérazinyl]]-2-phénylbutyryl]-1H-azépine racémique.

**2.** Procédé suivant la revendication 1, dans lequel le produit est l'énantiomère de la 2,3,4,5,6,7-hexahydro-1-[4-[1-[4-(2-méthoxyphényl)pipérazinyl]]-2-phénylbutyryl]-1H-azépine, pour lequel la base libre a un $[\alpha]_D^{26}$ d'environ +53° (1% dans le $CHCl_3$), ou un sel pharmaceutiquement acceptable de celui-ci.

**3.** Procédé suivant la revendication 1, dans lequel le produit est l'énantiomère de la 2,3,4,5,6,7-hexahydro-1-[4-[1-[4-(2-méthoxyphényl)pipérazinyl]]-2-phénylbutyryl]-1H-azépine, pour lequel la base libre a un $[\alpha]_D^{26}$ d'environ -62° (1% dans le $CHCl_3$), ou un sel pharmaceutiquement acceptable de celui-ci.

**4.** Procédé de préparation d'une composition pharmaceutique comprenant la mise en association de la 2,3,4,5,6,7-hexahydro-1-[4-[1-[4-(2-méthoxyphényl)pipérazinyl]]-2-phénylbutyryl]-1H-azépine ou d'un sel pharmaceutiquement acceptable de celle-ci avec un excipient pharmaceutiquement acceptable.

**5.** Procédé suivant la revendication 4, dans lequel l'ingrédient actif est préparé par le procédé suivant l'une quelconque des revendications 1 à 3.